# EUROPEAN PATENT SPECIFICATION

(11) **EP 2 468 349 B1**
(45) Date of publication and mention of the grant of the patent: **06.03.2019**
(21) Application number: 12161105.7
(22) Date of filing: 16.09.2005
(51) Int. Cl.: A61F 2/86, A61B 17/12, A61F 2/82

(54) **Thin film metallic devices for plugging aneurysms or vessels**
Dünnfilm-Metallvorrichtungen zum Stecken in Aneurysmen oder Gefäße
Dispositifs métalliques à film mince pour colmater des anévrismes ou des vaisseaux

(30) Priority: 17.09.2004 US 611016 P
(43) Date of publication of application: 27.06.2012
(62) Divisional of application: 05798100.3
(73) Proprietor: Codman & Shurtleff, Inc., Raynham, MA 02767 (US)
(72) Inventor: Slazas, Robert, Miami, FL Florida 33176 (US); Jones, Donald, Dripping Springs, TX Texas 78620 (US)
(74) Representative: Curran, Clair

(56) References cited:
- WO-A2-2004/019791

## Description

This invention generally relates to medical devices that are implantable within a vessel of a patient and that have occlusion capabilities that are especially suitable for use as medical device plugs for aneurysms or for defective or diseased body vessels. These types of devices have a shape which diverts blood flow away from aneurysms and a porosity that reduces or prevents blood from flowing into or out of an aneurysm.

Medical devices that can benefit from the present invention include those that are introduced endoluminally and expand when deployed so as to plug up a location of concern within the patient. These are devices that move between collapsed and expanded conditions or configurations for ease of deployment through catheters and introducers. The present disclosure focuses upon occlusion devices for aneurysms or other defects or diseased locations within the vasculature, explicitly including those that are sized, shaped and constructed for neurovascular use. An aneurysm is an abnormal bulge or ballooning of the wall of a blood vessel. Typically, an aneurysm develops in a weakened wall of an arterial blood vessel. The force of the blood pressure against the weakened wall causes the wall to abnormally bulge or balloon outwardly. One detrimental effect of an aneurysm is that the aneurysm may apply undesired pressure to tissue surrounding the blood vessel. This pressure can be extremely problematic, especially in the case of a cranial aneurysm where the aneurysm can apply pressure against sensitive brain tissue. Additionally, there is also the possibility that the aneurysm may rupture or burst, leading to more serious medical complications including mortality. When a patient is diagnosed with an unruptured aneurysm, the aneurysm is treated in an attempt to reduce or lessen the bulging and to prevent the aneurysm from rupturing. Unruptured aneurysms have traditionally been treated by what is commonly known in the art as "clipping." Clipping requires an invasive surgical procedure wherein the surgeon makes incisions into the patient's body to access the blood vessel containing an aneurysm. Once the surgeon has accessed the aneurysm, he or she places a clip around the neck of the aneurysm to block the flow of blood into the aneurysm which prevents the aneurysm from rupturing. While clipping may be an acceptable treatment for some aneurysms, there is a considerable amount of risk involved with employing the clipping procedure to treat cranial aneurysms because such procedures require open brain surgery. More recently, intravascular catheter techniques have been used to treat cranial aneurysms because such techniques do not require cranial or skull incisions, i.e., these techniques do not require open brain surgery. Typically, these techniques involve using a catheter to deliver embolic devices to a preselected location within the vasculature of a patient. For example, in the case of a cranial aneurysm, methods and procedures, which are well known in the art, are used for inserting and guiding the distal end of a delivery catheter into the vasculature of a patient to the site of the cranial aneurysm. A coil-like vascular occlusion device then is attached to the end of a pusher member which pushes the occlusion device through the catheter and out of the distal end of the catheter where the occlusion device is delivered into the aneurysm. Once the occlusion device has been deployed within the aneurysm, the blood clots on the occlusion device and forms a thrombus. The thrombus forms an occlusion which seals off the aneurysm, preventing further ballooning or rupture. In some instances, the deployment procedure is repeated until multiple coil-like occlusion devices are deployed within the aneurysm. With these aneurysm-packing approaches, typically, it is desired to deploy enough coil-like devices to obtain a packing density of about 20% or more, preferably about 35% and more if possible.

The most common coil-like vascular occlusion devices are embolic coils. Embolic coils typically are constructed from a metal wire which has been wound into a helical shape. One of the drawbacks of embolic coils for some applications is that they do not provide a large surface area for blood to clot thereto. Additionally, the embolic coil may be situated in such a way that there are relatively considerable gaps between the coil and the aneurysm wall or adjacent coils in which blood may freely flow. The addition of extra coils into the aneurysm does not always solve this problem because deploying too many coils into the aneurysm may lead to an undesired rupture. Therefore, there remains a need that is recognized and addressed according to the present invention for an occlusion device which can function alone in order to plug an entrance into an aneurysm or other vessel defect with the objective of enhancing the effectiveness of the occlusion device in stopping or severely restricting blood flow into the diseased space or aneurysm, without increasing the risk of rupturing the aneurysm. Examples of devices which follow a general approach of aneurysm plugging include that disclosed in US-6168622. Metal fabric strands are given a bulbous shape which is intended to occupy substantial space within the aneurysm, while an "anchor" is intended to hold the device in place. Strands of metals including nickel-titanium alloys generally known as "nitinol" metal alloys are proposed for making into metal fabric by braiding techniques. The occlusion capabilities of the braided metal are determined during the manufacturing process. One of the drawbacks associated with the known device is that when the device is implanted with a blood vessel of a patient, the device disrupts the normal laminar blood flow. This disruption causes an unnatural turbulent blood flow which may lead to undesired damage to the blood vessel. Technologies other than braiding have been used in the medical device field. These include using thin film technologies. Current methods of fabricating thin films (on the order of several µm thick) employ material deposition techniques. These methods are known to make films into basic shapes, such as by depositing onto a mandrel or core so as to make thin films having the shape of the mandrel or core, such as geometric core shapes until the desired amount has built up. Traditionally, a thin film is generated in a simple (oftentimes cylindrical, conical, or hemispherical) form and heat-shaped to create the desired geometry. One example of a known thin film vapour deposition process is disclosed in US-A-2005/0033418. Methods for manufacturing three-dimensional medical devices using planar films have been suggested, as disclosed in US-6746890. The method described in this document requires multiple layers of film material interspersed with sacrificial material. Accordingly, the methods described therein are time- consuming and complicated because of the need to alternate between film and sacrificial layers.

For some implantable medical devices, it is preferable to use a porous structure. Typically, the pores are added by masking or etching techniques or laser or water jet cutting. When occlusion devices are porous, especially for intercranial use, the pores are extremely small and these types of methods are not always satisfactory and can generate accuracy issues. Approaches such as those proposed by US-A-2003/0018381 include vacuum deposition of metals onto a deposition substrate which can include complex geometrical configurations. Microperforations are mentioned for providing geometric distendability and endothelization. Such microperforations are said to be made by masking and etching. An example of porosity in implantable grafts is disclosed in US-A-2004/ 0098094. This publication proposes endoluminal grafts having a pattern of openings, and indicates different orientations thereof could be practised. Underlying stents support a microporous metallic thin film. Also, US-5540713discloses apparatus for widening a stenosis in a body cavity by using a stent-type of device having slots which open into diamonds when the device is radially expanded. A problem to be addressed is to provide a plug- like occlusion device that can be delivered endoluminally in intercranial applications which provides an immediate occlusive function to "plug" the aneurysm or vessel defect and control or stop blood flow into the diseased site while diverting blood flow away from the aneurysm or other defective area in a manner that substantially maintains normal laminar blood flow.

WO2004/019791 describes a vascular occlusion device that includes an umbrella shaped occlusive member that expands from an unexpanded to an expanded state to close a vascular defect. The occlusive member can be secured to a stent by means of a securement member. The occlusive member can be made from a memory metal. The connector element is initially in an unexpanded state but when in place can be expanded to secure itself (and therefore the embolisation element) to struts of the stent. This can be achieved using an expanding gel or using arms or leaves that can bend around the stent struts in the side wall of the stent. The embolisation element, in the unexpanded state, is fed through the stent towards the vascular defect and inserted into the vascular defect through openings in the stent wall. The occlusion element is then expanded to close the vascular defect and the securing element is expanded to secure the occlusion element to the stent by attaching itself to the struts in the stent wall.

Accordingly, a general aspect or object of the present invention is to provide an occlusion device which performs a plugging function that greatly reduces or completely blocks the flow of blood into or out of an aneurysm.

In an aspect of the present invention there is provided a vascular occlusion device according to claim 1.

Another aspect or object of this invention is to provide a method for plugging an aneurysm or other vessel defect that can be performed in a single endoluminal procedure and that positions an occlusion device for effective blood flow blockage into the diseased location. Another aspect or object of this invention is to provide an improved occlusion device that incorporates thin film metal deposition technology in preparing neurovascular occlusion devices that divert the flow of blood away from an aneurysm while maintaining the normal laminar flow of blood.

Another aspect or object of the present invention is to provide an occlusion device having a three- dimensional configuration that has shape features set thereinto that form upon deployment and that are designed for plugging openings of diseased vasculature. Another aspect or object of this invention is to provide an occlusion system having an occlusion device that anchors in place after deployment by a member that is at a location external of the aneurysm or defect. Another aspect or object of the present invention is to provide an occlusion system having an occlusion device that diverts a substantial portion of the blood flow in the vicinity of the occlusion system to flow around the aneurysm or defect location.

Other aspects, objects and advantages of the present invention, including the various features used in various combinations, will be understood from the following description according to preferred embodiments of the present invention, taken in conjunction with the drawings in which certain specific features are shown.

In accordance with the present invention, occlusion devices are provided for treating a diseased vessel of a patient, and more particularly for treating an aneurysm. The invention is especially suitable for treating a distal basilar tip aneurysm. The occlusion device includes an embolisation element which is connected to an anchor element that aids in maintaining the embolisation element in place.

The invention is defined by the appended claims. Subject matter referred as embodiments and/or inventions which are not claimed are not part of the invention.

The embolisation element has a thin film structure that has a contracted or collapsed configuration which facilitates endoluminal deployment as well as an expanded or deployed configuration for plugging an aneurysm. When in the deployed configuration, the thin film of the embolisation element is shaped with a distal end of a larger cross-sectional extent when compared to the rest of the deployed device. Such deployed shapes can be generally funnelled in shape or hemispherically shaped. When the occlusion device is deployed, the embolisation element plugs an aneurysm by abutting the larger distal end of the embolisation element against a wall of an artery surrounding the outside of a neck of the aneurysm, or by placing the embolisation element within the aneurysm so that the proximal end of the embolisation element plugs the neck of the aneurysm. The porosity of the embolisation element is low enough to either substantially reduce or fully block the flow of blood into or out of the aneurysm. This causes the blood to stagnate within the aneurysm and form an occluding thrombus. Additionally, it is preferred that the shape of the embolisation element also substantially reduces turbulence and aids in maintaining a substantially laminar blood flow in the vicinity of the implanted device.

In making the thin film embolisation element, a core or mandrel is provided which is suited for creating a thin film by a physical vapour deposition technique, such as sputtering. A film material is deposited onto the core to form a seamless or continuous three-dimensional layer. The thickness of the film will depend on the particular film material selected, conditions of deposition and so forth. Typically, the core then is removed by chemically dissolving the core, or by other known methods. Manufacturing variations allow the forming of multiple layers of thin film material or a thicker layer of deposited material if desired.

An anchor element that is connected to the embolisation element by a connector element aids in retaining the embolisation element in place and reduces the risk of the embolisation element becoming dislodged and migrating to an undesired location. The anchor element is preferably a self expanding stent, but may also be a balloon expandable stent or any other suitable anchor member.

### BRIEF DESCRIPTION OF THE DRAWINGS

Fig. 1 is a front elevational view of an occlusion device according to an exemplary vascular occlusion device, in a collapsed configuration.
Fig. 2 is a front elevational view of the occlusion device of Fig. 1 in a deployed configuration.
Fig. 3 is perspective view of the occlusion device of Fig. 1 in a deployed configuration.
Fig. 4 is a front elevational view of another exemplary occlusion device in a deployed configuration.
Fig. 5 is an enlarged partial sectional view of the occlusion device of Fig. 1 and a delivery system disposed within a basil artery and aligned adjacent to a basilar tip aneurysm.
Fig. 6 is an enlarged partial sectional view of a deployment catheter moved proximally with the proximal section of an embolisation element of the occlusion device of Fig. 1 compressed within the deployment catheter and the distal section of the embolisation expanded into a deployed configuration.
Fig. 7 is an enlarged sectional view of the occlusion device of Fig. 1 implanted within a basil artery.
Fig. 8 is a front elevational view of another exemplary occlusion device in the collapsed configuration.
Fig. 9 is a front elevational view of the occlusion device of Fig. 8 in a deployed configuration.
Fig. 10 is a front elevational view of the occlusion device of the present invention in a deployed configuration.
Fig. 11 is an enlarged partial sectional view of the occlusion device of Fig. 8 and a delivery system disposed within a basil artery and aligned adjacent to a basilar tip aneurysm.
Fig. 12 is an enlarged partial sectional view of a deployment catheter moved proximally with the proximal section of an embolisation element of the occlusion device of Fig. 8 compressed within the deployment catheter and the distal section of the embolisation expanded into a deployed configuration within the aneurysm.
Fig. 13 is an enlarged sectional view of the occlusion device of Fig. 8 implanted within the vessel; and
Fig. 14 is an enlarged sectional view of another exemplary occlusion device implanted within a blood vessel that has a straight line relationship with an aneurysm.

### DESCRIPTION OF THE PREFERRED EMBODIMENTS

As required, detailed embodiments of the present invention are disclosed herein; however, it is to be understood that the disclosed embodiments are merely exemplary of the invention, which may be embodied in various forms. Therefore, specific details disclosed herein are not to be interpreted as limiting, but merely as a basis for the claims and as a representative basis for teaching one skilled in the art to variously employ the present invention in virtually any appropriate manner.

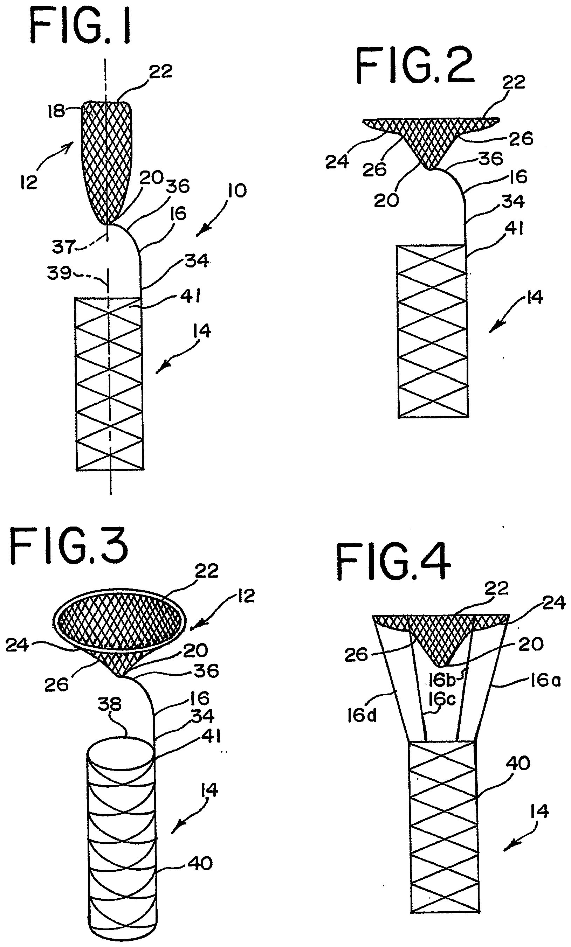

Fig. 1 shows a preferred example of an occlusion device of the present invention in the contracted or collapsed position. The occlusion device 10 comprises an embolisation element 12 attached to an anchor element 14 by a connector element 16. The embolisation element 12 preferably comprises a thin film formed by physical vapour deposition onto a core or mandrel, as is well-known to those skilled in the art. Most preferably, a thin film of a nitinol (which encompasses alloys of nickel and titanium), or other suitable material which has the ability to take on a shape that has been imparted to it during manufacture, is formed. When nitinol material, for example, is used in forming the thin film, the thin film can be at the martensite state. In addition, the thin film when made of nitinol or materials having similar shape memory properties may be austenite with a transition from martensite to austenite, typically when the device is raised to approximately human body temperature, or in the range of about 35°C (95°F) to 38°C (100°F).

In making the thin film, this selected material is sputter-deposited onto a core, which core is then removed by chemical etching or the like. Examples of this type of deposition are disclosed in US-A-2003/0018381, US-A-2004/0098094 and US-A-2005/ 0033418. Nitinol is a preferred film material because of its superelastic and shape memory properties, but other known biocompatible compositions with similar characteristics may also be used.

The thickness of the thin film layer depends on the film material selected, the intended use of the device, the support structure, and other factors. A thin film, such as a thin film of nitinol, is preferably between about 0.1 and 250 µm thick and typically between about 1 and 30 µm thick. More preferably, the thickness of the thin film is between about 1 and 10 µm or at least about 0.1 µm but less than about 5 µm. Supported films can be thinner than films that are self-supporting. The embolisation element 12 has a plurality of pores or openings 18 according to an aspect of the present invention. The pores 18 may be formed by any known means, but are preferably formed using laser-cutting. The illustrated pores 18 are shown in Fig. 1 with generally identical diamond-shaped openings which are arranged in a uniform pattern along the length of the embolisation device 12, but they may assume other open profiles and be arranged randomly or in selected non-uniform patterns, depending on the intended use.

The pores 18 serve at least two functions. First, the pores 18 aid in allowing the embolisation element 12 expand or transform into a deployed configuration, as illustrated in Fig. 2. Second, the pores 18 are sized so that blood flow through the embolisation element is greatly reduced or substantially blocked when the device is deployed.

The embolisation element 12 has a closed proximal end portion 20 and a distal end portion 22. In the illustrated embodiment, the distal end portion is generally open. In the collapsed configuration, the embolisation element 12 has a generally cylindrical shape and a reduced radial cross-section as compared to the deployed configuration. In the collapsed state the occlusion device 10 can be introduced to a site adjacent an aneurysm or other diseased or defective area through a delivery catheter.

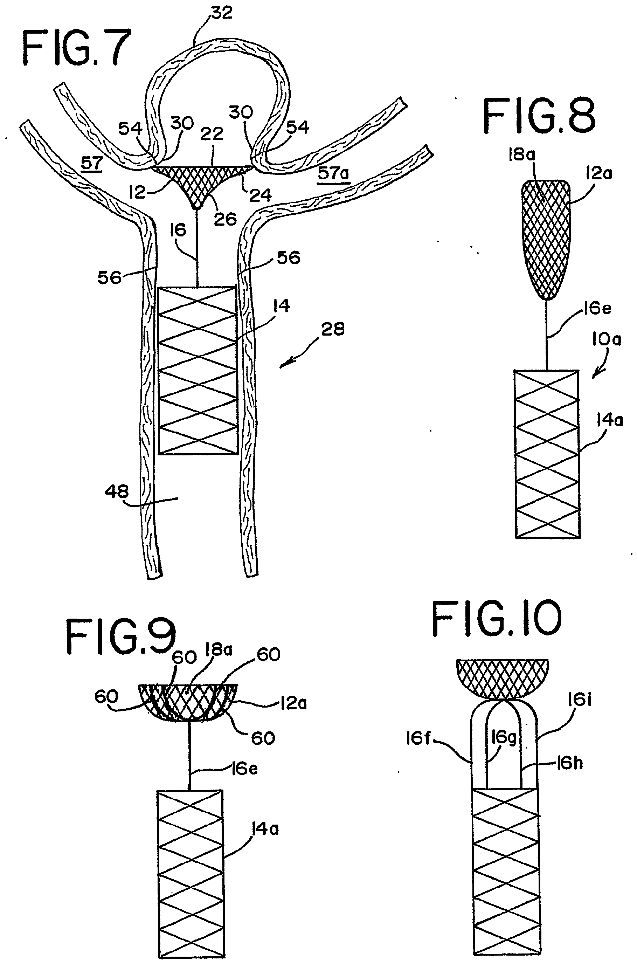

Referring to Figs. 2-4, in the deployed configuration, the embolisation element 12 is generally funnel shaped and the distal end portion 22 has a larger cross-sectional extent than the proximal end portion 20. Additionally, the outer surface 24 of the embolic element 12 has generally inwardly curved contour 26 that extends circumferentially around the embolisation element 12. The occlusion device 10 may be deployed within a basil artery 28 so that the distal end portion 22 of the embolisation element 12 covers the opening of the neck 30 of a basilar tip aneurysm 32, as illustrated in Fig. 7. The curved contour 26 of the outer surface 24 diverts the flow of blood away from the aneurysm 32 in a manner that reduces undesired turbulence and aids in maintaining normal laminar blood flow.

When the thin film of the embolisation element is comprised of a nitinol shape memory alloy or other similarly functional shape memory material, the embolisation element may be heat set to form the austenitic shape or deployed configuration of the embolisation element into a generally funnelled shape as illustrated in Figs. 2- 4. In the martensitic state, the thin film embolisation element 12 is preferably generally cylindrically shaped as illustrated in Fig. 1.

Referring to Figs. 1-3, the embolisation element 12 is connected to the anchor element 14 by at least one connector element 16 having a proximal end portion 34 and a distal end portion 36. As best seen in Fig. 3, the proximal end portion 34 of the connector element 16 is depicted as being connected to a rim 38 located at a distal end portion 41 of the anchor element 14, and the distal end portion 36 of the connector element 16 is connected to the closed ended proximal end portion 20 of the embolisation element 12. The connector element 16 preferably extends from the rim 38 of the anchor element 14 so that the proximal section of the connector element 16 substantially remains in the same plane as the wall of the anchor element. The distal end portion 36 of the connector element 16 is curved so that the longitudinal axis 37 of the embolisation element 12 is generally aligned with the longitudinal axis 39 of the anchor element 14. It is also contemplated that the respective longitudinal axes of the embolisation element and the anchor element need not be aligned with each other, depending on the desired use. Thus, the invention can find application in situations where the aneurysm or other defect is not in a straight-line relationship with the portion of the vessel within which the anchor element is implanted. Whatever its shape or location, a preferred feature of the connector element 16 is that it exhibit minimal interference with the blood flow by allowing the connector element to follow along the wall of the artery and avoid crossing the path of the blood flow. As illustrated in Fig. 4, more than one connecter element may be used to connect the embolisation element 12 to the anchor element 14. As illustrated, connector elements 16a, 16b, 16c and 16d may be used to connect the embolisation element 12 to the anchor element 16. Additionally, the connector elements 16a-d may be connected to the distal end portion 22 of the embolisation element 12 instead of the proximal end portion 20.

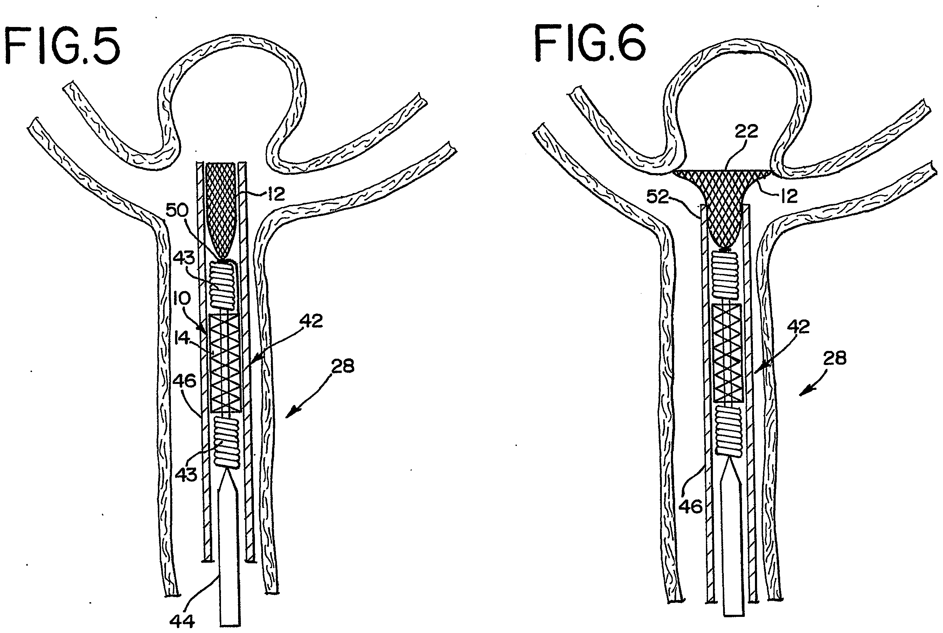

The connector element 16 is preferably comprised of a nitinol but may also be any other suitable material, such as biocompatible metals and polymers. The connecter element 16 may be connected to the anchor element and the embolisation element by weld, solder, adhesive or any other suitable manner that is in keeping with the biocompatibility requirements of implanted devices. The anchor element 14 preferably comprises an expandable stent 40 which may take on many different configurations and may be self-expandable or balloon expandable. Examples of such stents are disclosed in US-6673106 and US-6818013. Preferably, the expandable stent 40 is laser cut from a tubular piece of nitinol. When the occlusion device is deployed, the expandable stent 40 expands within the artery and aids in maintaining the embolisation element 12 in place. Fig. 5 illustrates the occlusion device 10 within a delivery system 42 position inside of a basil artery 28. An example of a delivery system that may be use to deploy the occlusion device 10 is disclosed in US-6833003. As illustrated, a pusher element 44 is used to push and guide the occlusion device 10 through a delivery catheter 46 which has been positioned within the main basil artery 48. The anchor element 14 is positioned between two cylindrical elements 43 and 43a of pusher element 44 until deployment. A distal end portion 50 of the pusher element 44 contacts the embolisation device 12 which may or may not be releasably attached to the distal end portion 50 of the pusher element 44. This arrangement allows the anchor element 14 and the embolisation element 12 to be guided through the delivery catheter.

Fig. 6 illustrates the expandable embolisation element 12 partially deployed within the basil artery 28. The deployment catheter 46 is moved proximally causing the distal end portion 22 of the embolisation element 12 to exit the distal end 52 of the delivery catheter 46 and partially deploy.

Fig. 7 illustrates the occlusion device 10 fully deployed within the basil artery 28 with the delivery system 42 removed. The distal end portion 22 of the expanded embolisation element 12 contacts the wall 54 of the artery 28 adjacent the neck 30 of the aneurysm 32 and substantially reduces blood flow into or out of the aneurysm. The anchor element 14 expands radially outwardly and contacts the wall 56 of the main artery 48 to anchor the occlusion device 10. The embolisation element 12 is held in place by the pressure of the blood flow pressing the embolisation element against the wall 54 of the artery 28. Additionally, the anchor element 14 in conjunction with the connector element 16 also aids in maintaining the embolisation element 12 in place and greatly reduces the risk of migration of the embolisation element 12 to an undesired location.

Once the occlusion device 10 is in the deployed position, the embolisation element 12 plugs the aneurysm 32 which causes the blood within the aneurysm to stagnate and form an occluding thrombus. The occluding thrombus within the aneurysm 32 greatly reduces the risk of a rupture of the aneurysm. Additionally, the generally funnel shaped embolisation element 12 redirects the blood flow away from the aneurysm 32 toward the branch arteries 57 and 57a while substantially maintaining laminar blood flow. Another embodiment of an occlusion device of the present invention is generally illustrated in Fig. 8. The vascular occlusion device 10a is similar to the previous embodiment in that the occlusion device includes an embolisation device 12a connected to an anchor element 14a via at least one connector element 16e. The embolisation element 12a, anchor element 14a and connector element 16a may be made from the same materials and assembled in the substantially the same manner as described above. Additionally, as illustrated in Fig. 10, the embolisation element 12a of the present invention is connected to the anchor element 14a by connector elements 16f, 16g, 16h and 16i instead of just a single connector element, as shown in Fig. 8 and Fig. 9.

In the contracted or collapsed state, the embolisation element 12a has generally cylindrical configuration, similar to that of the previous example. As illustrated in Fig. 9, in the deployed configuration, or the austenitic state when the embolisation element 12a is comprised of nitinol, the embolisation element 12a has a hemispherical shape.

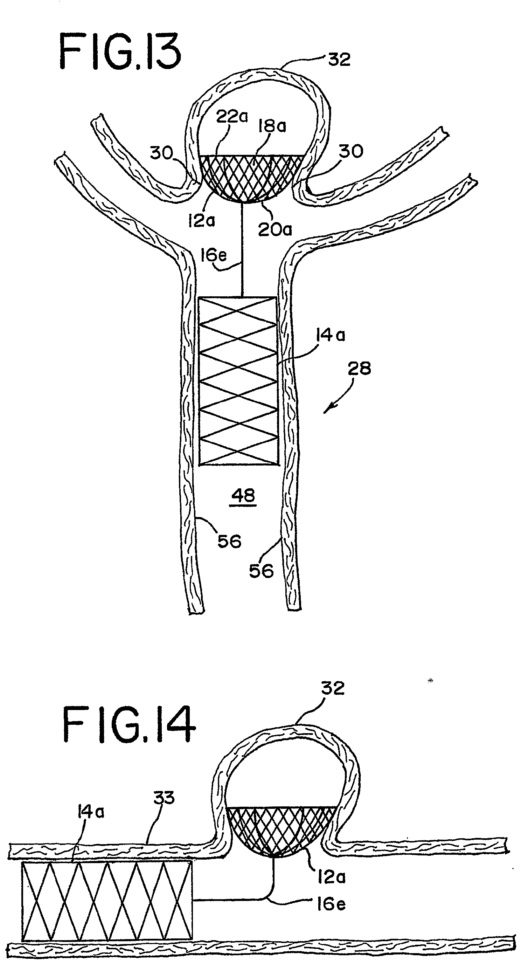

When deployed, the hemispherical embolisation element 12a is placed within the aneurysm 32 so that the proximal end portion 20a of the embolisation element 12a blocks the neck 30 of the aneurysm 32, as illustrated in Fig. 13. Similar to the previous example, the embolisation element 12a includes pores or apertures 18a in the thin film. The pores 18a are sized to greatly reduce or substantially block the flow of blood into the aneurysm 32 when the system is deployed within a living patient. The connector elements can be formed into different configurations depending upon the desired application of the occlusion device. For example, as illustrated in Fig. 14, the connector element 16e can be configured to accommodate situations where the aneurysm 32 or other defect is not in a straight-line relationship with the portion of the vessel 33 within which the anchor element 14a is implanted.

Referring back to Fig. 9, the embolisation element 12a may also include at least one support strut 60 which may be strands of material attached to the thin film of the embolisation device 12a. Alternatively, the struts maybe unitary with the thin film and formed during sputtering by methods of masking the core that are generally known to those in the art. The struts 60 provide support to the thin film so that a thinner film may be used, if desired.

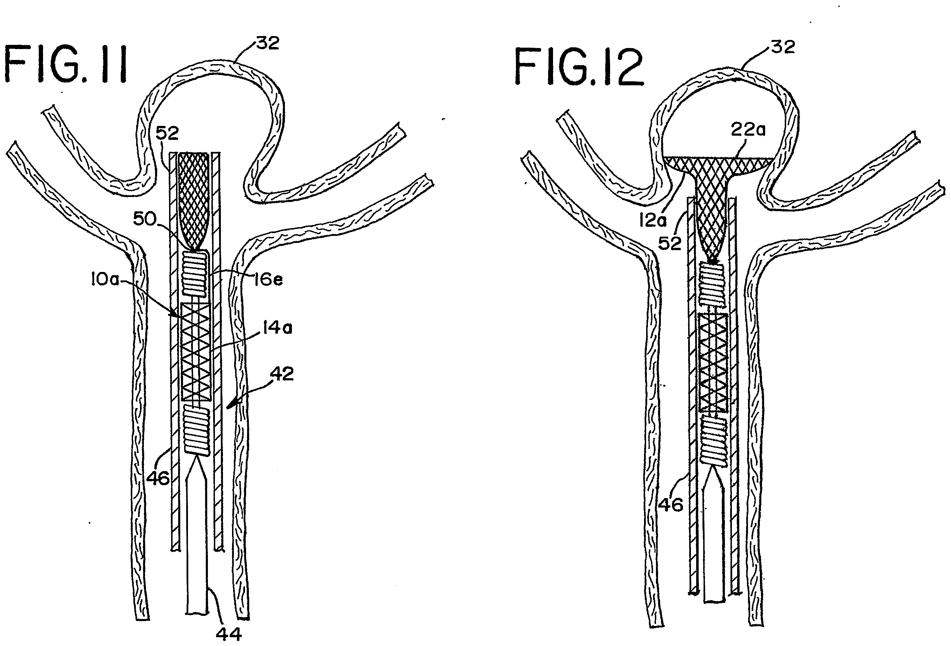

Fig. 11 illustrates the occlusion device 10a within a delivery system 42. A delivery catheter 46 is positioned so that the distal end portion 52 of the delivery catheter 46 extends to the location to be treated, typically into a basilar tip aneurysm 32. The pusher element 44 is used to push and guide the occlusion device 10a through a delivery catheter. The anchor element 14a is positioned and retained over a portion of a pusher element 44 and the distal end portion 50 of the pusher element contacts the embolisation device 12a, which may or may not be releasably attached to the distal end portion 50 of the pusher element 44.

Fig. 12 illustrates the expandable embolisation element 12a partially deployed within the aneurysm 32. The delivery catheter 46 is moved proximally causing the distal end portion 22a of the embolisation element 12a to exit the distal end 52 of the delivery catheter 46 and partially deploy. Fig. 13 illustrates the occlusion device 10a fully deployed within the basil artery 28 with the delivery system 42 removed. The expanded embolisation element 12a is deployed within the aneurysm 32 and the proximal end portion 20a of the embolisation element 12a plugs the neck 30 of the aneurysm 32 and substantially reduces blood flow into or out of the aneurysm. The anchor element 14a expands radially outwardly and contacts the wall 56 of the main artery 48 to anchor the occlusion device 10a in place. Additionally, the anchor element 14a in conjunction with the connector element 16a aids in maintaining the embolisation element 12a in place and greatly reduces the risk of migration of the embolisation element 12a to an undesired location.

It is also disclosed a vascular occlusion device comprising: an embolisation element comprised of a thin film of a shape memory alloy having a plurality of pores extending through the thin film; said embolisation element having a collapsed state and an expanded state; an anchor element for securing the embolisation element within a blood vessel of a patient; and at least one connector element connecting the embolisation element to the anchor element.

Optionally, the shape memory alloy is a nitinol.

Optionally, the shape memory alloy is transformable between an austenitic state and a martensitic state; and wherein the embolisation element is in the expanded position when the shape memory alloy is in the austenitic state and in the collapsed position when the shape memory alloy is in the martensitic state.

Optionally, the embolisation element is adapted to cover a neck of an aneurysm in the expanded position.

Optionally, the embolisation element is adapted to extend into an aneurysm in the expanded position.

Optionally, the embolisation element has a generally funnel-like shape in the expanded position.

Optionally, the embolisation element has a generally hemispherical shape in the expanded position.

Optionally, the embolisation element includes at least one support strut.

Optionally, the anchor element comprises a stent.

Optionally, the stent comprises a self-expanding stent.

Optionally, the thin film of shape memory alloy has a thickness greater than about 0.1 µm but less than about 5 µm.

It is also disclosed a vascular occlusion device comprising: an embolisation element comprised of a thin film of shape memory alloy having a plurality of pores extending through the thin film; said embolisation element has a collapsed state and an expanded state wherein the embolisation element assumes a generally funnel-shaped configuration in the expanded state; the generally funnel-shaped configuration of the embolisation element in the expanded state has a proximal end portion and a distal end portion that is sized and shaped to plug the neck of an aneurysm; an anchor element for securing the embolisation element within a blood vessel of a patient; and at least one connector element connecting the embolisation device to the anchor element.

Optionally, the vascular occlusion device of claim 1, wherein the shape memory alloy is a nitinol.

Optionally, the shape memory alloy is transformable between an austenitic state and a martensitic state; and wherein the embolisation element is in the expanded position when the shape memory alloy is in the austenitic state and in the collapsed position when the shape memory alloy is in the martensitic state.

Optionally, the connector element is connected to the proximal end portion of the embolisation element.

Optionally, the anchor element comprises a stent.

## Claims

1. A vascular occlusion device (10), which comprises:
an embolisation element (12) having a longitudinal axis (37) and comprising a thin film of shape memory alloy having a plurality of pores extending through the thin film, the embolisation element having a collapsed state and an expanded state in which the embolisation element assumes a generally hemispherically shaped configuration in the expanded state, the generally hemispherically shaped configuration of the embolisation element in the expanded state having a distal end portion and a closed proximal end portion that is sized and shaped to plug the neck of an aneurysm,
an anchor element (14), having a wall and a rim (38), and a longitudinal axis (39), for securing the embolisation element within a blood vessel of a patient, and
a plurality of connector elements (16f,16g,16h,16i) connecting the embolisation device to the anchor element, each connector element having a proximal end portion (34) and a distal end portion (36), curved so that the longitudinal axis of the embolisation element is generally aligned with the longitudinal axis of the anchor element, the proximal end portion being connected to the rim of the anchor element such that the proximal end portion substantially remains in the same plane as the wall of the anchor element, and the distal end being connected to the embolization element.

2. The vascular occlusion device of claim 1, in which the shape memory alloy is a nitinol.

3. The vascular occlusion device of claim 1, in which the shape memory alloy is transformable between an austenitic state and a martensitic state, and in which the embolisation element is in the expanded position when the shape memory alloy is in the austenitic state and in the collapsed position when the shape memory alloy is in the martensitic state.

4. The vascular occlusion device of claim 1, in which the embolisation element includes at least one support strut.

5. The vascular occlusion device of claim 1, in which the anchor element comprises a stent.

## Patentansprüche

1. Gefäßverschlussvorrichtung (10), umfassend:
ein Embolisierungselement (12), das eine Längsachse (37) aufweist und einen Dünnfilm aus Formgedächtnislegierung umfasst, der eine Vielzahl von Poren aufweist, die sich durch den Dünnfilm erstrecken, wobei das Embolisierungselement einen reduzierten Zustand und einen expandierten Zustand aufweist, wobei das Embolisierungselement im expandierten Zustand eine allgemein halbkugelförmige Ausgestaltung annimmt, wobei die allgemein halbkugelförmige Ausgestaltung des Embolisierungselements im expandierten Zustand einen distalen Endabschnitt und einen geschlossenen proximalen Endabschnitt aufweist, der bemessen und geformt ist, um in den Hals eines Aneurysmas gesteckt zu werden,
ein Ankerelement (14), das eine Wand und einen Rand (38) und eine Längsachse (39) zum Sichern des Embolisierungselements innerhalb eines Blutgefäßes eines Patienten aufweist, und
eine Vielzahl von Verbinderelementen (16f, 16g, 16h, 16i), die die Embolisierungsvorrichtung mit dem Ankerelement verbinden, wobei jedes Verbinderelement einen proximalen Endabschnitt (34) und einen distalen Endabschnitt (36) aufweist, die derart gekrümmt sind, dass die Längsachse des Embolisierungselements allgemein mit der Längsachse des Ankerelements ausgerichtet ist, wobei der proximale Endabschnitt derart mit dem Rand des Ankerelements verbunden ist, dass der proximale Endabschnitt im Wesentlichen in der gleichen Ebene bleibt wie die Wand des Ankerelements und das distale Ende mit dem Embolisierungselement verbunden ist.

2. Gefäßverschlussvorrichtung nach Anspruch 1, wobei die Formgedächtnislegierung ein Nitinol ist.

3. Gefäßverschlussvorrichtung nach Anspruch 1, wobei die Formgedächtnislegierung zwischen einem austenitischen Zustand und einem martensitischen Zustand unwandelbar ist, und wobei das Embolisierungselement sich in der expandierten Position befindet, wenn die Formgedächtnislegierung sich im austenitischen Zustand befindet, und sich in der reduzierten Position befindet, wenn die Formgedächtnislegierung sich in dem martensitischen Zustand befindet.

4. Gefäßverschlussvorrichtung nach Anspruch 1, wobei das Embolisierungselement mindestens eine Stützstrebe umfasst.

5. Gefäßverschlussvorrichtung nach Anspruch 1, wobei das Ankerelement einen Stent umfasst.

## Revendications

1. Dispositif d'occlusion vasculaire (10) qui comprend :
un élément d'embolisation (12) ayant un axe longitudinal (37) et comprenant un film fin d'alliage à mémoire de forme ayant une pluralité de pores s'étendant à travers le film fin, l'élément d'embolisation ayant un état replié et un état déployé dans lequel l'élément d'embolisation adopte une configuration de forme généralement hémisphérique à l'état déployé, la configuration généralement de forme hémisphérique de l'élément d'embolisation à l'état déployé ayant une partie d'extrémité distale et une partie d'extrémité proximale fermée qui est dimensionnée et formée pour boucher le col d'un anévrisme,
un élément d'ancrage (14) ayant une paroi et un bord (38), et un axe longitudinal (39) pour fixer l'élément d'embolisation à l'intérieur d'un vaisseau sanguin d'un patient, et
une pluralité d'éléments de connecteur (16f, 16g, 16h, 16i) raccordant le dispositif d'embolisation à l'élément d'ancrage, chaque élément de connecteur ayant une partie d'extrémité proximale (34) et une partie d'extrémité distale (36), incurvé de sorte que l'axe longitudinal de l'élément d'embolisation est généralement aligné avec l'axe longitudinal de l'élément d'ancrage, la partie d'extrémité proximale étant raccordée au bord de l'élément d'ancrage de sorte que la partie d'extrémité proximale reste sensiblement dans le même plan que la paroi de l'élément d'ancrage, et l'extrémité distale étant raccordée à l'élément d'embolisation.

2. Dispositif d'occlusion vasculaire selon la revendication 1, dans lequel l'alliage à mémoire de forme est un Nitinol.

3. Dispositif d'occlusion vasculaire selon la revendication 1, dans lequel l'alliage à mémoire de forme est transformable entre un état austénitique et un état martensitique et dans l'élément d'embolisation est dans la position déployée lorsque l'alliage à mémoire de forme est à l'état austénitique et dans la position repliée lorsque l'alliage à mémoire de forme est à l'état martensitique.

4. Dispositif d'occlusion vasculaire selon la revendication 1, dans lequel l'élément d'embolisation comprend au moins une entretoise de support.

5. Dispositif d'occlusion vasculaire selon la revendication 1, dans lequel l'élément d'ancrage comprend un stent.
